(19) Europäisches Patentamt

European Patent Office

Office européen des brevets

(11) **EP 1 085 914 B1**

(12) **EUROPÄISCHE PATENTSCHRIFT**

(45) Veröffentlichungstag und Bekanntmachung des Hinweises auf die Patenterteilung:
**16.11.2005 Patentblatt 2005/46**

(21) Anmeldenummer: **99927891.4**

(22) Anmeldetag: **05.06.1999**

(51) Int Cl.⁷: **A61L 15/44**, A61L 15/42, A61L 15/58

(86) Internationale Anmeldenummer:
**PCT/EP1999/003900**

(87) Internationale Veröffentlichungsnummer:
**WO 1999/064082 (16.12.1999 Gazette 1999/50)**

(54) **WIRKSTOFFHALTIGE PFLASTER**

PLASTER CONTAINING AN ACTIVE AGENT

EMPLATRE CONTENANT UN PRINCIPE ACTIF

(84) Benannte Vertragsstaaten:
**AT DE ES FR GB IT NL SE**

(30) Priorität: **08.06.1998 DE 19825499**

(43) Veröffentlichungstag der Anmeldung:
**28.03.2001 Patentblatt 2001/13**

(73) Patentinhaber: **Beiersdorf Aktiengesellschaft**
**20245 Hamburg (DE)**

(72) Erfinder:
• **BROSCHK, Katharina**
**CEP 12020-230 Taubaté, SP (BR)**
• **JAUCHEN, Peter**
**D-22455 Hamburg (DE)**
• **KÖHLER, Ulrich**
**D-22089 Hamburg (DE)**
• **HIMMELSBACH, Peter**
**D-21614 Buxtehude (DE)**
• **WASNER, Matthias**
**D-21029 Hamburg (DE)**

(56) Entgegenhaltungen:
EP-A- 0 053 936          EP-A- 0 914 820
DE-A- 19 620 109         FR-A- 1 421 732
US-A- 4 801 458          US-A- 5 660 854

## Beschreibung

Die Erfindung betrifft wirkstoffhaltige Pflaster bestehend aus einem Trägermaterial und einer zumindest partiell aufgebrachten Klebemasse, wobei die Klebemasse des Pflasters den Wirkstoff oder gegebenenfalls auch mehrere Wirkstoffe enthält, die an die Haut abgegeben werden, sowie Verfahren zur Herstellung der Pflaster.

Stark klebende Pflaster werden üblicherweise vollflächig mit einer Zink-Kautschuk-Klebemasse beschichtet. Das Verkleben derartiger Produkte auf der Haut zeigt nach dem Ablösen deutliche Hautirritationen und eine mechanische Beanspruchung der Haut. Die Verklebung läßt sich ohne Hilfsmittel nur unter Schmerzen lösen. Fallweise kommt es zu allergischen Reaktionen. Die verwendeten Klebemassen führen darüber hinaus oft zu einem Massetransfer, d.h. einem Umspulen, auf die Haut.

Die Verwendung von hautfreundlichen Klebemassen, wie Acrylatklebemassen und Hydrogele, ist aufgrund der geringen Scherstabilität und Anfaßklebrigkeit nicht erwägenswert. Eine Verbesserung durch eine Nachbehandlung, insbesondere Vernetzung, ist möglich, jedoch bleibt das Ergebnis insgesamt unbefriedigend. Die propriorezeptive Wirkung ist gegenüber den Systemen mit einer Harz-Kautschuk-Klebemasse geringer.

Andere bekannte Klebesysteme auf Basis von herkömmlichen Blockcopolymeren sind zum einen durch hohen Stabilisatorzusatz nicht hautfreundlich beziehungsweise zeigen durch die hohe Kohäsivität bislang nur eine Eignung für technische Anwendungsfälle, zum anderen sind sie nicht stark hautklebend und hauthaftend einzustellen.

Die zuvor genannten Klebemassen sind druckempfindliche Selbstklebemassen, wobei die Massen für die Verarbeitung in einer Trägermatrix vorliegen können. Als Trägermatrix werden gängige organische oder anorganische Lösemittel oder Dispergiermittel verstanden.

Systeme ohne Trägermatrix werden als 100 %-Systeme bezeichnet und sind ebenfalls nicht unbekannt. Sie werden im elastischen oder thermoplastischen Zustand verarbeitet. Eine gängige Verarbeitungsweise ist die Schmelze. Auch solche Haftschmelzklebemassen sind im Stande der Technik bereits vorbeschrieben. Sie basieren auf natürlichen oder synthetischen Kautschuken und/oder anderen synthetischen Polymeren.

Vorteilhaft an den 100 %-Systemen ist, dass bei ihnen verfahrenstechnisch ein Entfemen der Trägermatrix, d.h. der Hilfsmittel vermieden wird, wodurch sich die Verarbeitungsproduktivität steigert und sich gleichzeitig der Maschinen- und Energieaufwand reduziert. Weiter wird so ein Verbleiben von Reststoffen der Trägermatrix reduziert. Dieses begünstigt wiederum die Senkung des allergenen Potentials.

Aufgrund ihrer hohen Härte ist für solche 100 %-Systeme die Hauthaftung problematisch.

Es ist ferner bekannt, derartige Selbstklebemassen nicht nur vollflächig sondern auch rasterpunktförmig aufzubringen, beispielsweise durch Siebdruck (DE-PS 4237252), wobei die Klebstoffpünktchen auch unterschiedlich groß und/oder unterschiedlich verteilt (EP 0353972 B) oder durch Tiefdruck von in Längs- und Querrichtung zusammenhängenden Stegen (DE-PS 4308649) aufgebracht sein können.

Der Vorteil des rasterförmigen Auftrags besteht darin, dass die Klebematerialien bei entsprechend porösem Trägermaterial luft- und wasserdampfdurchlässig sowie in der Regel leicht wieder ablösbar sind.

Ein Nachteil dieser Produkte besteht jedoch in der Tatsache, dass bei zu hoher Flächendeckung der an sich undurchlässigen Klebeschicht die Luft- und Wasserdampfdurchlässigkeit sich entsprechend verringert, sowie der Klebemassenverbrauch steigt und bei geringer Flächendeckung der Klebeschicht die Klebeeigenschaften leiden, d.h., das Produkt löst sich, insbesondere bei schweren, textilen Trägermaterialien, zu leicht vom Untergrund.

Transdermale Therapeutische Systeme (TTS) sind Darreichungsformen von Medikamenten, die einen oder mehrere Arzneistoffe über einen definierten Zeitraum an ihrem Anwendungsort an die Haut abgeben. Es wird dabei zwischen systemisch und lokal wirksamen Darreichungsformen unterschieden. Bei systemisch wirkenden Darreichungsformen gelangt der Wirkstoff durch Diffusion durch die Haut in den Blutkreislauf und kann im ganzen Körper wirken.

Lokal wirkende Darreichungsformen wirken dagegen nur an den applizierten Stellen. Der Wirkstoff verbleibt in der Haut beziehungsweise in den darunter liegenden Schichten.

Im Stande der Technik sind bereits zahlreiche Ausführungsformen wirkstoffhaltiger Pflaster beschrieben worden, die zum Teil nach dem Reservoirprinzip arbeiten, bei welchem der Wirkstoff beispielsweise über eine Membran abgegeben wird, teilweise auch mit einem Matrixsystem oder mit komplizierterem mehrschichtigen Aufbau.

Es ist ferner bekannt, dass die Klebemasse des Pflasters als den Wirkstoff enthaltende Matrix eingesetzt werden kann. Neben aus Lösung aufgetragenen Selbstklebemassen werden auch schon Heißschmelzselbstklebemassen dafür vorgeschlagen, zum Beispiel in der EP 0 663 431 A, EP 0 452 034 A, EP 0 305 757 A, DE-OS 43 10 012, DE-OS 42 22 334 und DE-C 42 24 325. Als Wirkstoffe werden hierbei, wenn diese benannt werden, systemisch wirkende aufgeführt.

Beispielhaft für wirkstoffhaltige Pflaster seien die durchblutungsfördernden Wirkstoffpflaster genannt, die zu der Gruppe der lokal wirksamen therapeutischen Systeme gehören. Die Anwendung solcher Pflaster ist angezeigt zur Behandlung von rheumatischen Beschwerden, Ischias, Hexenschuß, Nackensteifigkeit, Schulter-Arm-Schmerzen sowie Muskelverspannungen und -zerrungen, Muskelkater oder Muskel-, Gelenk- und Nervenschmerzen im Bereich des Bewegungsapparates.

Capsaicin und Nonivamid sind bekannte Wirkstoffe solcher lokal, durchblutungsfördernd wirkender Pflaster. Aufgrund ihrer Anwendung am Bewegungsapparat müssen sie in der Regel stark kleben. Üblicherweise werden die Pflaster vollflächig mit einer Harz-Kautschuk-Klebemasse beschichtet, welche den Wirkstoff enthält.

Derartige Pflaster, welche meist größer flächig aufgebracht werden müssen, können zeigen jedoch nach dem Ablösen bei empfindlichen Patienten fallweise mechanische Hautirritationen verursachen. Ihr Entfemen ist nach längerer Tragezeit bis zu einem gewissen Grade schmerzhaft.

Ein weiterer Nachteil der bekannten wärmewirksamen Pflaster mit einer Klebemasse auf Basis von natürlichem Kautschuk, die in Form einer Lösung mit organischen Lösungsmitteln auf den Pflasterträger aufgetragen wird, ist die vergleichsweise niedrige Freisetzungsrate des Wirkstoffs.

Die geschilderten und weitere Nachteile treffen ebenso auch auf wirkstoffhaltige Pflaster zu, die andere als die genannten Stoffe beinhalten.

So beschreibt die WO 94/02123 ein Wirkstoffpflaster auf Basis von Haftschmelzklebemassen, welche niedrigschmelzende und/oder leicht flüchtige Wirkstoffe in einer Konzentration von 2,5 Gew.-% bis 25 Gew.-% enthält. Bei den dort eingesetzten Polymeren handelt es sich um A-B-A-Dreiblock Styrol-Ethylen-Butylen-Styrol Blockcopolymerisate, welche sich durch einen geringen Initialtack und geringe Klebkraft auf Haut auszeichnen. Die wirkstoffhaltige Komponente ist nicht versponnen.

EP 0 663 431 A2, EP 0 443 759 A3, EP 0 452 034 A2 und US 5,371,128, beschreiben Verwendungen von druckempfindlichen Schmelzhaftklebern auf Silikonbasis mit diversen Additiven und in differenzierten Aufbauformen. Die wirkstoffhaltige Komponente ist nicht versponnen.

DE 43 10 012 A1 beschreibt den Aufbau eines dermalen therapeutischen Systems aus schmelzfähigen Poly(meth)acrylat-Mischungen. Die wirkstoffhaltige Komponente ist nicht versponnen.

DE 43 16 751 C1 beschreibt ein Mehrkammersystem zur Darreichung von Wirkstoffen. Die wirkstoffhaltige Komponente ist nicht versponnen.

EP 0 439 180 beschreibt ein Wirkstoffpflaster zur Darreichung von Tulobuterol. Die wirkstoffhaltige Komponente ist nicht versponnen.

EP 0 305 757 beschreibt ein Wirkstoffpflaster zur Darreichung von Nicotin. Die wirkstoffhaltige Komponente ist nicht versponnen.

EP 0 305 758 beschreibt ein Wirkstoffpfiaster zur Darreichung von Nitroglycerin. Die wirkstoffhaltige Komponente ist nicht versponnen.

EP 0 305 756 beschreibt eine Vorrichtung von Stoffen und ihrer Herstellung und Verwendung. Die wirkstoffhaltige Komponente ist nicht versponnen.

DE 37 43 945 beschreibt eine Vorrichtung zur Abgabe von Stoffen sowie das Herstellverfahren. Bei der beschriebenen Haftschmelzklebemasse auf Basis von SIS handelt es sich um keine selbstklebende Vorrichtung. Die dort angegebenen Verarbeitungsbereiche liegen deutlich unter denen von Heißschmelzklebemassen und würden für solche beschriebenen Systeme keine ausreichende Verankerung der Klebemasse bieten. Die wirkstoffhaltige Komponente ist nicht versponnen.

WO 96/22083 zeigt einen Polyisobutylenkleber für transdermale Zwecke, welcher einen Klebrigmacher mit hohem Glasübergangspunkt hat. Die wirkstoffhaltige Komponente ist nicht versponnen.

US 4801458 offenbart wirkstoffhaltige Pflaster bestehend aus einem Trägermaterial und einer zumindest partiell aufgebrachten Klebemasse, wobei die Klebemasse eine zumindest teilweise thermoplastische Basis aufweisen kann. Das Pflaster besteht aus drei Komponenten, dem Träger, der Klebemasse und wirkstoffhaltigen hohlen Fasern in Form von gewebten und gewirkten Gewebe. Die den Wirkstoff enthaltenen Fasern kommen zudem nicht in Kontakt mit der Haut.

DE 19620109 und auch FR 1421732 offenbaren eine Auswahl an möglichen Klebemassen, die zum Teil eine thermoplastische Basis aufweisen und ggf. geschäumt sind aber wirkstofffrei sind.

Aufgabe der Erfindung war es, wirkstoffhaltige Pflaster zur Verfügung zu stellen, welche sich bei Vermeidung der aus dem Stand der Technik bekannten Nachteile durch eine gute Wirksamkeit, d.h. eine relativ hohe Freisetzungsrate, und gute Hautverträglichkeit bei gleichzeitig gutem Klebeverhalten auszeichnen. Auch sollen sie sich technisch wenig aufwendig und umweltverträglich herstellen lassen.

Gelöst wird diese Aufgabe durch wirkstoffhaltige Pflaster gemäß dem Hauptanspruch. Gegenstand der Unteransprüche sind vorteilhafte Ausführungsformen der erfindungsgemäßen Pflaster. Des weiteren umfasst die Erfindung Verfahren zur Herstellung derartiger Pflaster.

Demgemäß betrifft die Erfindung wirkstoffhaltige Pflaster bestehend aus einem Trägermaterial und einer zumindest partiell aufgebrachten Klebemasse, die sich dadurch auszeichnen, dass

    a) auf dem Trägermaterial eine einen oder mehrere Wirkstoffe enthaltene Klebemasse in Form von versponnenen Fasern oder Fäden aufgebracht ist,

b) die versponnene Faser oder der versponnene Faden eine Dicke von weniger als 300 µm aufweist und

c) die Klebemasse eine zumindest teilweise thermoplastische Basis aufweist.

Vorzugsweise liegen die Mengenkonzentrationen des oder der Wirkstoffe in der Klebemasse zwischen 0,01 bis ca. 60 Gew.-%, besonders vorzugsweise 0,1 bis 50 Gew.-%. Unter der Bezeichnung "Wirkstoffe" im Zusammenhang mit der vorliegenden Erfindung werden chemische Elemente, organische und anorganische Verbindungen, die aus den sie enthaltenden Bestandteilen einer gattungsgemäßen Vorrichtung herauswandern können und dadurch einen angestrebten Effekt hervorrufen, verstanden. Unter den Anwendungsgebieten der erfindungsgemäßen Vorrichtung ist die Human- und Tiermedizin von besonderer Bedeutung, wobei hier eine Ausgestaltung der Erfindung in Pflasterform besonders bevorzugt ist.

Typische, über erfindungsgemäß hergestellte Vorrichtungen verabreichbare Stoffe sind hierbei: Aceclidin, Amfetaminil, Amfetamin, Amylnitrit, Apophedrin, Atebrin, Alpostadil, Azulen, Arecolin, Anethol, Amylenhydrat, Acetylcholin, Acridin, Adenosintriphosphorsäure, L-Äpfelsäure, Alimemazin, Allithiamin, Allyl Isothiocyanat, Aminoethanol, Apyzin, Apiol, Azatadin, Alprenolol, Äthinazon, Benzoylperoxid, Benzylalkohol, Bisabolol, Bisnorephedrin, Butacetoluid, Benactyzin, Campher, Colecalciferol, Chloralhydrat, Clemastin, Chlorobutanol, Capsaicin, Cyclopentamin, Clobutinol, Chamazulen, Dimethocain, Codein, Chloropromazin, Chinin, Chlorthymol, Cyclophosphamid, Cinchocain, Chlorambuzil, Chlorphenesin, Diethylethan, Divinylethan, Dexchlorpheniramin, Dinoproston, Dixyrazin, Ephedrin, Ethosuximid, Enallylpropymal, Emylcamat, Erytroltetranitrat, Emetin, Enfluran, Eucalyptol, Etofenamat, Ethylmorphin, Fentanyl, Fluanison, Guajazulen, Halothan, Hyoscyamin, Histamin, Fencarbamid, Hydroxycain, Hexylresorcin, Isoaminilcitrat, Isosorbiddinitrat, Ibuprofen, Jod, Jodoform, Isoaminil, Lidocain, Lopirin, Levamisol, Methadon, Methyprylon, Methylphenidat, Mephenesin, Methylephedrin, Meclastin, Methopromazin, Mesuximid, Nicethamid, Norpseudoephedrin, Menthol, Methoxyfluran, Methylpentinol, Metixen, Mesoprostol, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäureester, Spartein, Trichlorethylen, Timolol, Trifluperazin, Tetracain, Trimipramin, Tranylcypromin, Trimethadion, Tybamat, Thymol, Thioridazin, Valproinsäure, Verapamil sowie weitere, dem Fachmann geläufige, über die Haut, eingeschlossen Schleimhäute, aufnehmbare Wirkstoffe. Selbstverständlich ist diese Aufzählung nicht abschließend.

Die Verteilung der Wirkstoffe in der Klebemasse erfolgt vorzugsweise in einem Thermohomogenisator, wie zum Beispiel Thermomixer, Thermokneter, Walzenwerke oder Schneckensysteme. Die Zugabe des Wirkstoffs kann in die vollständig hergestellte Klebemasse erfolgen. Beispielhaft kann der Wirkstoff auch in eine Zwischenstufe oder in die Ausgangsmischung eingearbeitet werden.

Vorteilhafterweise weist die versponnene Faser oder der versponnene Faden eine Dicke zwischen 0,1 bis 250 µm auf.

In einer bevorzugten Ausführungsform wird die Klebemasse geschäumt und weist einen Schäumungsgrad von mindestens 5 Vol.-% auf, vorzugsweise 10 Vol.-% bis 75 Vol.-%, besonders bevorzugt zwischen 40 Vol.-% und 60 Vol.-%.

Des weiteren hat es sich als besonders vorteilhaft herauskristallisiert, wenn die wirkstoffhaltige Klebemasse eine Selbstklebemasse ist. Die Klebemasse kann dabei identisch mit der ansonsten verwendeten Klebemasse sein.

Als Klebemassen für die wirkstoffhaltige Komponente und die ansonsten verwendete lassen sich vorteilhafterweise thermoplastische Heißschmelzselbstklebemassen einsetzen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich.

Ihr Erweichungspunkt sollte höher als 50 °C liegen, da die Applikationstemperatur in der Regel mindestens 90 °C beträgt, bevorzugt zwischen 120 °C und 150°C beziehungsweise 180 °C und 220 °C bei Silikonen. Gegebenenfalls kann eine Nachvernetzung durch UV- oder Elektronenstrahlen-Bestrahlung angebracht sein.

Insbesondere Heißschmelzselbstklebemassen auf Basis von Blockcopolymeren zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet.

Die hohe Scherfestigkeit der Heißschmelzselbstklebemasse wird durch die hohe Kohäsivität des Polymeren erreicht. Die gute Anfaßklebrigkeit ergibt sich durch die eingesetzte Palette an Klebrigmachern und Weichmachern.

Für besonders starkklebende Systeme basiert die Heißschmelzselbstklebemasse bevorzugt auf Blockcopolymeren, insbesondere A-B-, A-B-A-Blockcopolymere oder deren Mischungen. Die harte Phase A ist vornehmlich Polystyrol oder dessen Derivate, und die weiche Phase B enthält Ethylen, Propylen, Butylen, Butadien, Isopren oder deren Mischungen, hierbei besonders bevorzugt Ethylen und Butylen oder deren Mischungen.

Polystyrolblöcke können aber auch in der weichen Phase B enthalten sein, und zwar bis zu 20 Gew.-%. Der gesamte Styrolanteil sollte aber stets niedriger als 35 Gew.-% liegen. Bevorzugt werden Styrolanteile zwischen 5 Gew.-% und 30 Gew.-%, da ein niedrigerer Styrolanteil die Klebemasse anschmiegsamer macht.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

In einer vorteilhaften Ausführung weist die Heißschmelzselbstklebemasse die nachfolgend angegebene Zusammensetzung auf:

| 10 Gew.-% bis 90 Gew.-% | Blockcopolymere, |
|---|---|
| 5 Gew.-% bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher, |
| weniger als 15 Gew.-% | Additive, |
| weniger als 5 Gew.-% | Stabilisatoren, |
| weniger als 60 Gew.-% | Wirkstoff oder Wirkstoffe, insbesondere |
| 10 Gew.-% | Wirkstoff oder Wirkstoffe. |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Ein Füllen der Klebemasse mit mineralischen Füllstoffen, Fasern. Mikrohohl- oder -vollkugeln ist möglich.

Die Heißschmelzselbstklebemassen weisen insbesondere einen Erweichungspunkt auf oberhalb von 50 °C, bevorzugt zwischen 70 °C bis 220 °C, ganz besonders bevorzugt zwischen 75 °C bis 140 °C.

Die Heißschmelzselbstklebemassen sind vorzugsweise so eingestellt, dass sie bei einer Frequenz von 0,1 rad/s eine dynamisch-komplexe Glasübergangstemperatur von weniger als 15 °C, bevorzugt von 6 °C bis -30 °C, ganz besonders bevorzugt von 3 °C bis -25 °C, aufweisen.

Insbesondere an Pflaster werden hohe Anforderungen bezüglich der Klebeeigenschaften gestellt. Für eine ideale Anwendung sollte die Heißschmelzselbstklebemasse eine hohe Anfaßklebrigkeit besitzen. Die funktionsangepaßte Klebkraft auf der Haut und auf der Trägerrückseite sollte vorhanden sein. Weiterhin ist, damit es zu keinem Verrutschen kommt, eine hohe Scherfestigkeit der Heißschmelzselbstklebemasse notwendig.
Durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut und der Trägerrückseite erreicht.
Die hohe Scherfestigkeit der hier eingesetzten Selbstklebemasse wird durch die hohe Kohäsivität des Blockcopolymeren erreicht. Die gute Anfaßklebrigkeit ergibt sich durch die eingesetzte Palette an Klebrigmachern und Weichmachern.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet.
Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur die Heißschmelzselbstklebemasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient ($Q = \tan \delta$) zwischen dem Verlustmodul (G" viskoser Anteil) und dem Speichermodul (G' elastischer Anteil) ermittelt.

$$Q = \tan \delta = G''/G'$$

Für das subjektive Empfinden der Anfaßklebrigkeit (Tack) wird eine hohe Frequenz gewählt sowie für die Scherfestigkeit eine niedrige Frequenz.
Eine hoher Zahlenwert bedeutet eine bessere Anfaßklebrigkeit und eine schlechtere Scherstabilität.

Der komplexe-dynamische Glasübergangspunkt ist der Übergangspunkt vom amorphen in den viskoelastischen Bereich. Er entspricht dem Maximum der Temperaturfunktion bei vorgegebener Frequenz.

| Bezeichnung | $T_G$ niedrige Frequenz | Anschmiegsamkeit niedrige Frequenz/RT | Anfaßklebrigkeit hohe Frequenz/RT |
|---|---|---|---|
| Heißschmelzselbstklebemasse A | -12 ± 2 °C | tan δ = 0.08 ± 0,03 | tan δ = 0,84 ± 0,03 |
| Heißschmelzselbstklebemasse A | -9 ± 2 °C | tan δ = 0,22 ± 0,03 | tan δ = 1,00 ± 0,03 |

Bevorzugt werden erfindungsgemäß Heißschmelzselbstklebemassen, bei denen das Verhältnis des viskosen Anteils zum elastischen Anteil bei einer Frequenz von 100 rad/s bei 25 °C größer 0,6 ist, oder Heißschmelzselbstklebemassen, bei denen das Verhältnis des viskosen Anteils zum elastischen Anteil bei einer Frequenz von 0,1 rad/s bei 25 °C kleiner 0,6 ist, bevorzugt zwischen 0,35 und 0,02, ganz besonders bevorzugt zwischen 0,3 und 0,1.

Als bevorzugtes Verfahren zur erfindungsgemäß offenporigen Beschichtung luft- und wasserdampfdurchlässiger dotierter Klebeschichten kann die Spinntechnologie zur Applikation von thermoplastischen Kunststoffen und Klebern herangezogen werden.

Das kontaktlose Beschichten und Auftragen von Heißschmelzselbstklebemassen durch Spinnen mit Druckluft oder inerten Gasen findet bereits vielfach Einsatz.

Bei hohen Anforderungen an Gleichmäßigkeit und geringes Flächengewicht zeigen sich bei hohen Viskositäten jedoch enge Grenzen für die Beschichtung auf. Besonders bei Applikationen von höherviskosen Klebstoffen über 10 Pa*s tritt eine Neigung zum starken "Klecksen" auf. Aufgrund wissenschaftlicher Grundlagenforschung zur Spinnstrahltheorie wurden deshalb spezielle Spinnverfahren für höherviskose (hochmolekulare) Thermoplaste entwickelt.

Mit dem Meltspin-, Acufiber- und Durafiber-Verfahren sind heute Thermoplaste auf dem Pressur-Sensitivsektor bis 2000 Pa*s bei 200 °C verarbeitbar. Damit wird eine Breite von Einsatzmöglichkeiten auf dem Sektor des Spinnens von thermoplastischen Klebstoffen möglich.

Die Vorteile dieser Auftragstechnik wie berührungsloses, geometrieunabhängiges Beschichten bei gleichzeitiger geringer Wärmebelastung der Träger eröffnet völlig neue Pflasterspezialitäten.

Die Vielzahl der Verfahren werden beispielhaft von Acumeter, J+M-Laboratories, Dynafiber, ITO Dynatex Nordson beschrieben. Allen gleich sind die Strömungsvorgänge in den Spinndüsen. Die geschmolzenen thermoplastischen Kunststoffe zählen dabei zur Gruppe der nichtnewtonschen Flüssigkeiten mit strukturviskosem Fließverhalten, d.h., Schubspannung und Geschwindigkeit der Beschichtung sind nicht linear miteinander verbunden. Die als Schmelzklebstoffe gebräuchlichsten thermoplastischen Polymere bestehen aus linearen und/oder verzweigten Kettenmolekülen. Beim Vorgang des aerodynamischen Verstreckens wächst die Fließgeschwindigkeit am Spinnfaden und die Moleküle werden mehr und mehr entflochten und orientieren sich in der Strömungsrichtung.

Aus diesem Grunde haben alle Düsen ein Innenmischprinzip vorgesehen, bei dem die Klebstoffe innerhalb der Düsenkammer schon von strömender Druckluft umflossen werden. Der engste Spalt ist damit der Düsenaustritt. Dabei kann der Austrittsspalt rund oder schlitzförmig sein. Wird dieser Spinnfaden auf ein Substrat gesprüht, so entsteht ein sogenanntes Wirrvlies, dessen Struktur in sich verknüpft ist. Das Wirrvlies besteht aus einer regellosen, homogenen Anordnung eines verschlungenen Endlosfadens.

Die vliesartig versponnenen, über eine beliebig von der Beschichtungsbreite abhängende Anreihung von Düsen kontaktlos aufgetragene dotierte Kleberschicht verfügt über eine im Vergleich zu vollflächigen Beschichtungen vielfach höhere freie Oberfläche. Im Kontakt mit der Haut sind hier andere Liberationseigenschaften beziehungsweise Freisetzungskinetiken der jeweiligen Wirkstoffe und gegebenenfalls Wirkstoffkombinationen zu erwarten.

Die gezielte Beeinflussung beziehungsweise Veränderung/Erhöhung der Liberationseigenschaften durch vliesartig versponnene dotierte Schmelzhaftkleberbeschichtungen ist zu erwarten, da die vollflächigen dotierten Klebermatrices ein im allgemeinen diffusionsgesteuertes Freisetzungsverhalten aufweisen.

Im folgenden sollen anhand mehrerer Figuren Vorrichtungen zur Herstellung erfindungsgemäßer Pflaster dargestellt werden.

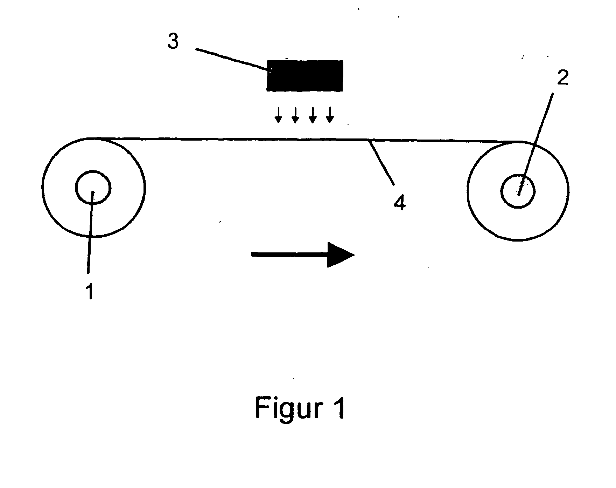

Im einfachsten Fall nach Figur 1 besteht die Vorrichtung aus einer Ab- (1) und Aufrollung (2) sowie der Trägerbahn (4) und der Spindüse (3).

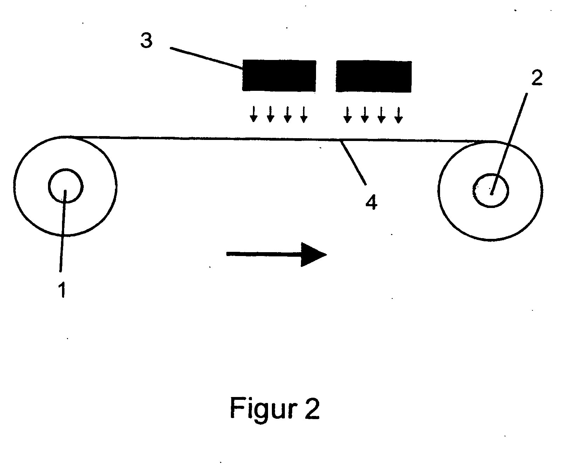

Zur Optimierung der Eigenschaften können gemäß Figur 2 auch mehrere Düsen in Reihe geschaltet werden.

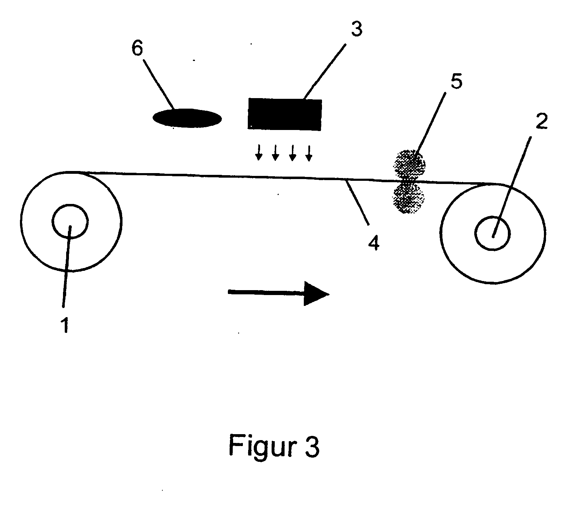

Für spezielle Träger kann eine Corona Vorbehandlung (6) oder nachträgliche Kalanderstation in den Versuchsaufbau mit aufgenommen werden, wie es in Figur 3 gezeigt ist.

Netzartig versponnene dotierte Schichten werden durch ihre offene Struktur eine eher oberflächengesteuerte Liberation aufweisen, insbesondere wenn durch körpereigene oder fremde Feuchtigkeit eine innige Benetzung der Kontaktflächen erfolgen kann.

Die Wechselwirkung der Wirkstoffe mit der Haut wird bekanntlich mit in die Klebemasse eingemischten Enhancem moduliert beziehungsweise durch den okklusiven Effekt von Klebemasse und Abdeckung verstärkt. Im Gegensatz hierzu kann durch den Einsatz von atmungsaktiven dotierten Beschichtungen in Verbindung mit elastischen ebenfalls atmungsaktiven Trägermaterialien ein insbesondere zum Beispiel während sportlicher Betätigungen

a) vom Anwender subjektiv angenehmer empfundener Tragekomfort und
b) vom Freisetzungsverhalten durch die mit der Umgebung ungestörtere Wechselwirkung (zum Beispiel Unterdrückung von Körperschweiß) der Haut eine definiertere Penetration von Wirkstoffen in die Haut erzielt werden.

Umgekehrt läßt sich durch die hier genannten Verfahren auch eine Durchlässigkeit des dotierten Pflastersystems von außen erzielen. Mit dieser Produkteigenschaft können somit von außen durch den Träger Substanzen an die Kontaktstelle dotierter Kleber/Haut auch zu späteren Zeiten nach der eigentlichen Applikation herangebracht werden (Flüssigkeit aufträufeln, wischen, etc.). Diese Substanzen könnten zum Beispiel eine zusätzliche Enhancerwirkung beinhalten oder die medikamentöse Wirkung starten, schwächen oder für eine entsprechende Konsumentenauslobung geeignet modulieren.

Ergänzend hierzu bietet auch das Spinnverfahren ebenfalls einige verfahrenstechnische Möglichkeiten für den Einsatz von partiellen Beschichtungen dotierter Schmelzhaftkleber:

- Durch Selektion einzelner Düsen in der quer zum Träger angeordneten Reihe von Auftragsdüsen kann nur der Wirkstoff mit einem geeigneten Trägermedium gezielt aufgebracht werden. Dies hat insofern den Vorteil, dass eventuelle Inkompatibilitäten eines Wirkstoffes mit einer Klebematrix vermieden werden und ein dotiertes Pflaster durch den erst zu einem sehr späten Zeitpunkt unmittelbar während des Beschichtungsvorganges nach Klebkraft und medikamentöser Wirkung optimiert werden kann.
- Der Auftrag von Wirkstoffen kann prinzipiell auch auf einen schon mit einer Klebeschicht versehenen Träger kontaktlos und partiell aufgebracht werden, wobei man hier frei in der Wahl des Trägers und der Beschichtungsform (partiell/vollflächig) ist.
- Die Trennung der Beschichtungsorgane in wirkstoffberührende und sonstige Anlagenkomponenten bietet zusätzlich den Vorteil einer GMP-gerechten Konfiguration der Beschichtungsanlage. Dies wird ermöglicht durch ein modulares Auswechseln und separates Reinigen nur begrenzter Anlagenteile (zum Beispiel Einsatz von Einzeldüsen als sogenanntes "dedicated equipment")

In einer besonderen Ausführung ist die Klebemasse geschäumt. Dieses muß für spezielle Anwendungen die funktionsgerechte Verwendung der Pflaster sicherzustellen.
Das mit den Wirkstoffen versehenen Klebemassecompound wird dabei bevorzugt mit inerten Gasen wie Stickstoff, Kohlendioxid, Edelgasen, Kohlenwasserstoffen oder Luft oder deren Gemischen geschäumt. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.
Der Schäumungsgrad, d.h. der Gasanteil, sollte mindestens 5 Vol.-% betragen und kann bis zu etwa 85 Vol.-% reichen. In der Praxis haben sich Werte von 10 Vol.-% bis 75 Vol.-%, bevorzugt 50 Vol.-%, gut bewährt. Wird bei relativ hohen Temperaturen von ungefähr 140 °C und vergleichsweise hohem Innendruck gearbeitet, entstehen sehr offenporige Klebstoffschaumschichten, die besonders gut luft- und wasserdampfdurchlässig sind.
Die vorteilhaften Eigenschaften der erfindungsgemäßen Klebebeschichtungen, hohe Anfaßklebrigkeit und gute Anschmiegsamkeit auch an unebenen Flächen durch die Elastizität und Plastizität der geschäumten Klebemassen sowie der Initialtack lassen sich auf dem Gebiet der Wirkstoffpflaster optimal nutzen.
Gleichzeitig wird durch die Vakuolen im Schaum der Transport bestimmter Wirkstoffe überproportional gesteigert, womit sehr gute Freisetzungsraten erreicht werden.
Ein besonders geeignetes Verfahren zur Herstellung der erfindungsgemäß geschäumten Klebemasse arbeitet nach dem Schaum-Mix-System. Hierbei wird die thermoplastische Klebemasse unter hohem Druck bei einer Temperatur über dem Erweichungspunkt (ungefähr 120 °C) mit den vorgesehenen Gasen wie zum Beispiel Stickstoff, Luft oder Kohlendioxid in unterschiedlichen Volumenanteilen (etwa 10 Vol.-% bis 80 Vol.-%) in einem Stator/Rotorsystem umgesetzt.
Während der Gasvordruck größer 100 bar ist, betragen die Mischdrucke Gas/Thermoplast im System 40 bis 100 bar, bevorzugt 40 bis 70 bar. Der so hergestellte Haftklebeschaum kann anschließend über eine Leitung in das Auftragswerk gelangen. Bei dem Auftragswerk finden handelsübliche Düsen, Sieb- oder Kammersysteme Verwendung.
Das geschäumte Klebemassecompound wird dann über das oben beschriebene Verfahren auf ein Trägermaterial appliziert. Hier erreicht man eine offenporigen Beschichtung, geregelt luft- und wasserdampfdurchlässige dotierter Schichten, welche geeigneterweise über zum Beispiel das Melt-spin-Verfahren oder Dura-fiber-Verfahren hergestellt werden.
Durch die Schäumung des Klebemassecompounds wird die benötigte Auftragsmenge erheblich reduziert

EP 1 085 914 B1

ohne nachteilige Beeinträchtigung der sonstigen Eigenschaften.

Durch das Schäumen wird zudem die Viskosität der Klebemassen in der Regel gesenkt. Hierdurch wird die Schmelzenergie emiedrigt, und es können auch thermoinstabile Trägermaterialien direkt beschichtet werden.

Vorzugsweise ist die wirkstoffhaltige Klebemasse mit einem Flächengewicht von größer 15 g/m$^2$, bevorzugt zwischen 30 g/m$^2$ und 500 g/m$^2$, ganz besonders bevorzugt zwischen 90 g/m$^2$ und 400 g/m$^2$, auf dem Trägermaterial aufgetragen.

Als Trägermaterialien eignen sich alle starren und elastischen Flächengebilde aus synthetischen und natürlichen Rohstoffen. Bevorzugt sind Trägermaterialien die nach Applikation der Klebemasse so eingesetzt werden können, dass sie Eigenschaften eines funktionsgerechten Verbandes erfüllen. Beispielhaft sind Textilien wie Gewebe, Gewirke, Gelege, Vliese, Laminate, Netze, sowie Folien, Schäume und Papiere aufgeführt. Weiter können diese Materialien vorbeziehungsweise nachbehandelt werden. Gängige Vorbehandlungen sind Corona und Hydrophobieren; geläufige Nachbehandlungen sind Kalandern, Tempern, Kaschieren, Stanzen und Eindecken und Vemetzen.

Je nach Trägermaterial und dessen Temperaturempfindlichkeit kann die geschäumte Heißschmelzselbstklebemasse direkt aufgetragen werden oder zuerst auf einen Hilfsträger aufgebracht und dann auf den endgültigen Träger transferiert werden.

Die zusätzlich zu der Klebemasse aufgebrachte Klebemasse wird vorteilhafterweise partiell auf dem Trägermaterial aufgetragen, insbesondere durch Rasterdruck, Siebdruck, speziell Thermosiebdruck, Thermoflexodruck oder Tiefdruck, speziell Gravurdruck oder durch Aufsprühen.

Der prozentuale Anteil der mit der Klebemasse beschichteten Fläche soll mindestens 10 % beträgt, bevorzugt 40 % bis 60 %, ganz besonders bevorzugt 70 % bis 95 %.

Auch ein nachträgliches Kalandem des beschichteten Produktes und/oder eine Vorbehandlung des Trägers, wie Coronabestrahlung, zur besseren Verankerung der Klebeschicht kann vorteilhaft sein.

Weiterhin kann eine Behandlung der geschäumten Heißschmelzselbstklebemasse mit einer Elektronenstrahl-Nachvernetzung oder einer UV-Bestrahlung zu einer Verbesserung der gewünschten Eigenschaften führen.

Das mit der versponnene Polymermasse beschichtete Trägermaterial kann eine Luftdurchlässigkeit von größer 1 cm$^3$/(cm$^{2*}$s), bevorzugt größer 15 cm$^3$/(cm$^{2*}$s), ganz besonders bevorzugt größer 70 cm$^3$/(cm$^{2*}$s), aufweisen, des weiteren eine Wasserdampfdurchlässigkeit von größer 500 g/(m$^{2*}$24h), bevorzugt größer 1000 g/(m$^{2*}$24h), ganz besonders bevorzugt größer 2000 g/(m$^{2*}$24h).

Schließlich kann das Material nach dem Beschichtungsvorgang mit einem abweisenden Trägermaterial, wie silikonisiertes Papier, eingedeckt oder mit einer Wundauflage oder einer Polsterung versehen werden.

Anschließend werden die Pflaster in der gewünschten Größe ausgestanzt.

Besonders vorteilhaft ist, dass das Material sterilisierbar, bevorzugt γ-(gamma) sterilisierbar, ist. So sind besonders geeignet für eine nachträgliche Sterilisation Heißschmelzselbstklebemassen auf Blockcopolymerbasis, welche keine Doppelbindungen enthalten. Dieses gilt ins besondere für Styrol-Butylen-Ethylen-Styrol-Blockcopolymerisate oder Styrol-Butylen-Styrol-Blockcopolymerisate. Es treten hierbei keine für die Anwendung signifikanten Änderungen in den Klebeeigenschaften auf.

Das erfindungsgemäße Pflaster kann eine Klebkraft auf der Trägerrückseite von mindestens 0,5 N/cm aufweisen, besonders eine Klebkraft zwischen 1,0 N/cm und 6,0 N/cm. Auf anderen Untergründen können höhere Klebkräfte erreicht werden.

Im folgenden sollen besonders vorteilhafte Pflaster der Erfindung beschreiben werden, ohne die Erfindung dadurch unnötig einschränken zu wollen.

**Beispiel**

Erfindungsgemäß wurde eine Vorrichtung zur Freigabe von Stoffen hergestellt, welche einen hyperämisieren Wirkstoff enthält. Die Vorrichtung kann aufgrund ihrer nachfolgend beschriebenen Eigenschaften zur Anwendung als Rheumapflaster dienen, welches auch aufgrund der guten klebetechnischen Eigenschaften mehrtägig an Gelenken des menschlichen Bewegungsapparates zu applizieren ist.

Das Trägermaterial bestand aus einem unelastischen Baumwollgewebe mit einer Höchstzugkraft von größer 80 N/cm und einer Höchstzugkraft-Dehnung von kleiner 20%.

Diese Schmelzhaftklebemasse setzte sich wie folgt zusammen:

- ein A-B/A-B-A Blockcopolymer, welches aus harten und weichen Segmenten besteht, mit einem Verhältnis der A-B-A zur A-B von 3:7 und einem Styrolgehalt im Polymer von 30 Mol.-%; der Anteil an der Klebemasse beträgt 32 Gew.-% (Kraton G)
- ein Paraffinkohlenwasserstoffwachs mit einem Anteil an der Klebemasse von 52 Gew.-%
- Kohlenwasserstoffharze mit einem Anteil von 14,5 Gew.-% (Super Resin HC 140)
- ein Alterungsschutzmittel mit einem Anteil von weniger als 0,5 Gew.-% (Irganox 1010)

-   ein hyperämisierender Wirkstoff (Nonanonyl Vanillylamid) mit einem Anteil von 1 %

Die eingesetzten Kleberkomponenten wurden in einem Thermomischer bei 185 °C 3h homogenisiert. Der Wirkstoff wurde in der Abkühlphase bei 140°C zugegeben und weitere 40 min im Mischer homogenisiert. Der Erweichungspunkt dieser Klebemasse betrug ca. 80 °C (DIN 52011), und die Klebemasse zeigte eine Viskosität von 2100 mPas bei 150 °C (DIN 53018, Brookfield DV II, Sp. 21). Die Glasübergang betrug nach o.g. Methode - 9 °C. Die so hergestellte dotierte Heißschmelzklebemasse wurde mit einer Dynafiber-Spray- düse auf den Träger appliziert. Die direkte Beschichtung erfolgte mit 10 m/min bei einer Temperatur von 120 °C. Das Trägermaterial wurde mit 170 g/m$^2$ beschichtet.

Das so hergestellte Pflastermaterial zeigt eine gute Freisetzung (Liberationsstudie) des Wirkstoffs. Nach 24-stündiger in vitro-Anwendung an Schweinehaut wurden 15% der Pflasterladung dermal absorbiert.

Die Luftdurchlässigkeit des offenporigen ausgerüsteten Trägermaterials betrug 25 cm$^3$/(cm$^2$*s). Nach der Applikation wurden keine Hautirritationen festgestellt.

**Patentansprüche**

1.  wrkstoffhaltige Pflaster bestehend aus einem Trägermaterial und einer zumindest partiell aufgebrachten Klebemasse, **dadurch gekennzeichnet, dass**

    a) auf dem Trägermaterial eine einen oder mehrere Wirkstoffe enthaltende Klebemasse in Form von versponnenen Fasern oder Fäden aufgebracht ist,
    b) die versponnene Faser oder der versponnene Faden eine Dicke von weniger als 300 μm aufweist und
    c) die Klebemasse eine zumindest teilweise thermoplastische Basis aufweist.

2.  Wirkstoffhaltige Pflaster gemäß Anspruch 1, **dadurch gekennzeichnet, dass** die Klebemasse den Wirkstoff in einer Menge von 0,01 bis 60 Gew.-%, bevorzugt von 0,1 bis 50 Gew.-%, enthält.

3.  Wirkstoffhaltige Pflaster nach den Ansprüchen 1 und 2, **dadurch gekennzeichnet, dass** die versponnene Faser oder der versponnene Faden eine Dicke zwischen 0,1 bis 250 μm aufweist.

4.  Wirkstoffhaltige Pflaster nach den Ansprüchen 1 bis 3, **dadurch gekennzeichnet, dass** die Klebemasse geschäumt ist und einen Schäumungsgrad von mindestens 5 Vol.-% aufweist, vorzugsweise 10 Vol.-% bis 75 Vol.- %, besonders bevorzugt zwischen 40 Vol.-% und 60 Vo).-%.

5.  Wirkstoffhaltige Pflaster gemäß mindestens einem der vorhergehenden Ansprüche, **dadurch gekennzeichnet, dass** die Klebemasse eine Heißschmelzselbstklebemasse auf Basis von synthetischen Kautschuken wie Blockcopolymeren, Acrylaten, Methacrylaten. Polyurethanen, Polyolefinen, Polyvinylderivaten, Polyacrylamiden, Polyestern oder Silikonen aufgebaut ist.

6.  Wirkstoffhaltige Pflaster gemäß Anspruch 5, **dadurch gekennzeichnet, dass** die Heißschmelzselbstklebemasse auf Blockcopolymerbasis aufgebaut ist, insbesondere A-B- oder A-B-A-Blockcopolymere oder deren Mischungen, wobei Phase A vomehmlich Polystyrol oder dessen Derivate und Phase B Ethylen, Propylen, Butylen, Butadien, Isopren oder deren Mischungen, hierbei besonders bevorzugt Ethylen und Butylen oder deren Mischungen, sind.

7.  Wirkstoffhaltige Pflaster gemäß mindestens einem der vorhergehenden Ansprüche, **dadurch gekennzeichnet, dass** die Heißschmelzselbstklebemasse enthält

| a) | 10 Gew.-% bis 90 Gew.-% | Blockcopolymere, |
|---|---|---|
| b) | 5 Gew.-% bis 80 Gew.-% | Klebrigmacher wie Öle, Wachse, Harze und/oder deren Mischungen, bevorzugt Mischungen aus Harzen und Ölen, |
| c) | weniger als 60 Gew.-% | Weichmacher, |
| d) | weniger als 15 Gew.-% | Additive, |
| e) | weniger als 5 Gew.-% | Stabilisatoren, |
| f) | 10 Gew.-% | Wirkstoff oder Wirkstoffe. |

8.  Wirkstoffhaltige Pflaster gemäß mindestens einem der vorhergehenden Ansprüche, **dadurch gekennzeichnet,**

**dass** die wirkstoffhaltige Klebemasse mit einem Flächengewicht von größer 15 g/m², bevorzugt zwischen 30 g/m² und 500 g/m², ganz besonders bevorzugt zwischen 90 g/m² und 400 g/m², auf dem Trägermaterial aufgetragen ist.

9.  Wirkstoffhaltige Pflaster gemäß mindestens einem der vorhergehenden Ansprüche, **dadurch gekennzeichnet, dass** die aufgebrachte Klebemasse partiell auf dem Trägermaterial aufgetragen wird, insbesondere durch Rasterdruck, Siebdruck, speziell Thermosiebdruck, Thermoflexodruck oder Tiefdruck, speziell Gravurdruck oder durch Aufsprühen.

10. Wirkstoffhaltige Pflaster gemäß mindestens einem der vorhergehenden Ansprüche, **dadurch gekennzeichnet, dass** der prozentuale Anteil der mit der Klebemasse beschichteten Fläche mindestens 10 % beträgt, bevorzugt 40 % bis 60 %, ganz besonders bevorzugt 70 % bis 95 %.

11. Wirkstoffhaltige Pflaster gemäß mindestens einem der vorhergehenden Ansprüche, **dadurch gekennzeichnet, dass** das Pflaster sterilisierbar, bevorzugt γ (gamma)-sterilisierbar, ist.

12. Wirkstoffhaltige Pflaster gemäß mindestens einem der vorhergehenden Ansprüche, erhalten nach dem Spinnverfahren wie Melt-Spin-Verfahren und/oder dem Dura-fiber-Verfahren.

**Claims**

1.  Active substance plasters consisting of a backing material and an at least partially applied adhesive composition, **characterized in that**

    a) an adhesive composition comprising one or more active substances is applied in the form of spun fibres or filaments to the backing material,
    b) the spun fibre or the spun filament has a thickness of less than 300 $\mu$m, and
    c) the adhesive composition has an at least partly thermoplastic basis.

2.  Active substance plasters according to Claim 1, **characterized in that** the adhesive composition comprises the active substance in an amount of from 0.01 to 60% by weight, preferably from 0.1 to 50% by weight.

3.  Active substance plasters according to Claims 1 and 2, **characterized in that** the spun fibre or the spun filament has a thickness of between 0.1 and 250 $\mu$m.

4.  Active substance plasters according to Claims 1 to 3, **characterized in that** the adhesive composition is foamed and has a degree of foaming of at least 5% by volume, preferably from 10 to 75% by volume and, with particular preference, between 40 and 60% by volume.

5.  Active substance plasters according to at least one of the preceding claims, **characterized in that** the adhesive composition is a hotmelt self-adhesive composition composed on the basis of synthetic rubbers such as block copolymers, acrylates, methacrylates, polyurethanes, polyolefins, polyvinyl derivatives, polyacrylamides, polyesters or silicones.

6.  Active substance plasters according to Claim 5, **characterized in that** the hotmelt self-adhesive composition is composed on the basis of block copolymers, especially A-B or A-B-A block copolymers or mixtures thereof, phase A being primarily polystyrene or its derivatives and phase B being ethylene, propylene, butylene, butadiene, isoprene or mixtures thereof, in which case particular preference is given to ethylene and butylene or their mixtures.

7.  Active substance plasters according to at least one of the preceding claims, **characterized in that** the hotmelt self-adhesive composition comprises

| a) | from 10 to 90% by weight | of block copolymers, |
|---|---|---|
| b) | from 5 to 80% by weight | of tackifiers such as oils, waxes, resins and/or mixtures thereof, preferably mixtures of resins and oils, |
| c) | less than 60% by weight | of plasticizers, |

(continued)

|  | d) | less than 15% by weight | of additives, |
|  | e) | less than 5% by weight | of stabilizers, and |
|  | f) | 10% by weight | of active substance or substances. |

8. Active substance plasters according to at least one of the preceding claims, **characterized in that** the adhesive composition comprising active substance is applied with a weight per unit area of more than 15 g/m$^2$, preferably between 30 and 500 g/m$^2$ and, with very particular preference, between 90 and 400 g/m$^2$, to the backing material.

9. Active substance plasters according to at least one of the preceding claims, **characterized in that** the applied adhesive composition is applied partially to the backing material, in particular by means of halftone printing, screen printing, especially thermal screen printing, thermal flexographic printing or intaglio printing, especially gravure printing, or by spraying.

10. Active substance plasters according to at least one of the preceding claims, **characterized in that** the percentage area that is coated with the adhesive composition is at least 10%, preferably from 40 to 60% and, with particular preference, from 70 to 95%.

11. Active substance plasters according to at least one of the preceding claims, **characterized in that** the plaster is sterilizable, preferably sterilizable by means of γ (gamma) radiation.

12. Active substance plasters according to at least one of the preceding claims, obtained by the spinning process such as melt-spin techniques and/or the Dura-fiber technique.


**Revendications**

1. Emplâtre contenant une ou plusieurs substances actives constitué par un matériau support et une masse adhésive appliquée au moins partiellement, **caractérisé**

   a) en ce qu'on applique, sur le matériau support, une masse adhésive contenant une ou plusieurs substances actives sous forme de fibres filées ou de brins filés,
   b) en ce que les fibres filées ou les brins filés présentent une épaisseur inférieure à 300 μm et
   c) en ce que la masse adhésive présente au moins partiellement une base thermoplastique.

2. Emplâtre contenant une ou plusieurs substances actives selon la revendication 1, **caractérisé en ce que** la masse adhésive contient la substance active en une quantité de 0,01 à 60% en poids, de préférence de 0,1 à 50% en poids.

3. Emplâtre contenant une ou plusieurs substances actives selon les revendications 1 et 2, **caractérisé en ce que** les fibres filées ou les brins filés présentent une épaisseur de 0,1 à 250 μm.

4. Emplâtre contenant une ou plusieurs substances actives selon les revendications 1 à 3, **caractérisé en ce que** la masse adhésive est moussée et présente un degré de moussage d'au moins 5% en volume, de préférence de 10% en volume à 75% en volume, de manière particulièrement préférée entre 40% en volume et 60% en volume.

5. Emplâtre contenant une ou plusieurs substances actives selon au moins l'une quelconque des revendications précédentes, **caractérisé en ce que** la masse adhésive est une masse auto-adhésive thermofusible élaborée à base de caoutchoucs synthétiques tels que les copolymères à blocs, les acrylates, les méthacrylates, les polyuréthanes, les polyoléfines, les dérivés de polyvinyle, les polyacrylamides, les polyesters ou les silicones.

6. Emplâtre contenant une ou plusieurs substances actives selon la revendication 5, **caractérisé en ce que** la masse auto-adhésive thermofusible est élaborée à base de copolymères à blocs, en particulier de copolymères à blocs A-B ou A-B-A ou leurs mélanges, la phase A étant essentiellement le polystyrène ou ses dérivés et la phase B l'éthylène, le propylène, le butylène, le butadiène, l'isoprène ou leurs mélanges, en préférant en particulier l'éthylène et le butylène ou leurs mélanges.

**7.** Emplâtre contenant une ou plusieurs substances actives selon au moins l'une quelconque des revendications précédentes, **caractérisé en ce que** la masse auto-adhésive thermofusible contient

a) 10% en poids à 90% en poids de copolymères à blocs,
b) 5% en poids à 80% en poids d'agents qui rendent adhésif, tels que les huiles, les cires, les résines et/ou leurs mélanges, de préférence les mélanges de résines et d'huiles,
c) moins de 60% en poids de plastifiants,
d) moins de 15% en poids d'additifs,
e) moins de 5% en poids de stabilisateurs,
f) 10% en poids de substance active ou de substances actives.

**8.** Emplâtre contenant une ou plusieurs substances actives selon au moins l'une quelconque des revendications précédentes, **caractérisé en ce que** la masse adhésive contenant une ou plusieurs substances actives est appliquée sur le matériau support en un poids surfacique supérieur à 15 g/m$^2$, de préférence entre 30 g/m$^2$ et 500 g/m$^2$, de manière tout particulièrement préférée entre 90 g/m$^2$ et 400 g/m$^2$.

**9.** Emplâtre contenant une ou plusieurs substances actives selon au moins l'une quelconque des revendications précédentes, **caractérisé en ce que** la masse adhésive appliquée est appliquée partiellement sur le matériau support, en particulier par impression matricielle, par sérigraphie, en particulier par thermosérigraphie, par thermoflexographie ou par héliographie, en particulier par gravure ou par pulvérisation.

**10.** Emplâtre contenant une ou plusieurs substances actives selon au moins l'une quelconque des revendications précédentes, **caractérisé en ce que** la proportion en % de la surface revêtue par la masse adhésive est d'au moins 10%, de préférence de 40% à 60%, de manière tout particulièrement préférée de 70% à 95%.

**11.** Emplâtre contenant une ou plusieurs substances actives selon au moins l'une quelconque des revendications précédentes, **caractérisé en ce que** l'emplâtre est stérilisable, de préférence par des rayons γ (gamma).

**12.** Emplâtre contenant une ou plusieurs substances actives selon au moins l'une quelconque des revendications précédentes, obtenu selon le procédé de filage, tel que le procédé de filature en fusion et/ou le procédé à fibres Dura.

Figur 1

Figur 2

Figur 3